# EUROPEAN PATENT APPLICATION

(11) **EP 0 772 226 A2**
(43) Date of publication of application: **07.05.1997**
(21) Application number: 96308014.8
(22) Date of filing: 05.11.1996
(51) Int. Cl.: H01J 65/04, C12H 1/16, B41M 7/00, A23C 3/07

(54) **Ultraviolet irradiating apparatus and components thereof**

(30) Priority: 06.11.1995 GB 9522686
(71) Applicant: R.A. Jenton & Co. Limited, Cheam, Surrey SM2 7BE (GB)
(72) Inventor: Little, Richard Anthony Rudd, Freemantle, Southampton SO 15 3BS (GB); Briggs, David, Silchester, Reading, Berkshire RG7 2HL (GB)
(74) Representative: Mackenzie, Andrew Bryan

(57) **Abstract**

Ultraviolet irradiating apparatus includes a microwave source and power supply (12,14) which energises electrodeless discharge lamps (8) to cause articles (4) travelling along conveyer (2) to be irradiated with ultraviolet energy. The lamps (8) may be partially inserted into a microwave cavity (10) as shown or may be coupled to a waveguide or coaxial line. The lamps (8) may include an antenna (34,42,48) arranged to equalise the intensity of ultraviolet emission through the volume of the lamp when the lamp is energised from an end. The apparatus may be used, for example, for sterilising, sanitising or ultraviolet curing.

## Description

The present invention relates to ultraviolet irradiating apparatus, to an electrodeless discharge lamp operable to emit ultraviolet radiation and to a microwave coupling for a coaxial line or a waveguide.

It has been established that ultraviolet light has the ability to breakdown DNA in bacteria at a wavelength of about 260 nm thereby impeding its reproduction cycle and to kill bacteria directly at a wavelength of about 190 nm. This makes ultraviolet radiation suitable for use in sterilisation and sanitisation processes. However, hitherto, limitations in the technology available to produce ultraviolet radiation has restricted its use in these fields.

To date, many sterilisation or sanitisation processes using ultraviolet radiation have employed medium pressure ultraviolet arc lamps as an ultraviolet source. These lamps are used so that the intensity of the ultraviolet radiation is sufficient to allow the sterilisation or sanitisation process to proceed at an economical speed. However, this type of lamp cannot be used for the sterilisation of a container since the electrical connections shadow parts of the inside of the container from the radiation. Moreover, the diameter of the bulb often is too large for the neck of the container. Since ultraviolet radiation is significantly attenuated by the materials from which most containers are manufactured, unless the lamp can be introduced into the interior of the container, sterilisation using this type of lamp is at best uneconomical and at worst ineffective. Similar problems occur when these lamps are used for curing, for example, of UV curable inks.

Other processes use a fluorescent tube adapted to radiate in the ultraviolet spectrum. Such tubes suffer from the same dimensional and shadowing problems as arc lamps, furthermore, radiate at lower powers (making sterilization or sanitization slower) and actually have a worse shadowing problem than arc lamps.

In the sterilisation and sanitation fields, alternatives to irradiation with ultraviolet light exist. The main alternatives are chemical sterilisation and sterilisation using high temperatures (typically steam sterilisation in an autoclave). The first of these alternatives requires a rinsing step following sterilisation particularly where containers are to be used for cosmetics or foodstuffs. This adds expense and complication. Furthermore a drying step is frequently also required. The second alternative is unsuitable for heat sensitive materials e.g. many plastics materials and also may require a drying step.

One aspect of the invention provides ultraviolet irradiating apparatus comprising a microwave source, an ultraviolet electrodeless discharge lamp and conveyor means for conveying articles to be irradiated along an irradiating path through the apparatus on a continuous basis, the lamp being mounted such that reciprocal movement of the conveyor means and/or the lamp relative to one another permit the lamp to be inserted and removed from articles passing along the irradiating path thereby to irradiate the articles with ultraviolet radiation emitted from the lamp. Preferably the apparatus is arranged such that the articles are not exposed to significant levels of microwave energy.

Thus all of the above problems are solved since microwave lamps generate no shadows since they have no electrical connections, they may be of much smaller diameter than similarly specified arc lamps and they may be started and stopped instantaneously and repeatedly without degradation of performance. Furthermore, since the apparatus may be arranged such that articles are not exposed to significant levels of microwave energy, the articles may be manufactured from a microwave sensitive material and the apparatus is well suited to continuous operation since the articles do not have to pass through a microwave cavity with the attendant difficulties in minimising leakage of microwave energy. The apparatus is particularly suited to a sterilisation or sanitisation process.

The apparatus may include a plurality of lamps and these may either be inserted partially into a microwave cavity or a rectangular waveguide with the portion of the or each lamp extending from the cavity being the portion which is inserted into the articles to be sterilised or the or each lamp may be directly coupled to a coaxial line carrying microwave energy. These arrangements allow the lamp to be energised from an end i.e. from the portion inserted into the cavity or the waveguide or from the portion coupled to the coaxial line.

Advantageously, the coaxial line may be flexible so that the microwave source may be statically mounted so that reciprocal movement of the lamp does not necessarily require corresponding reciprocal movement of the microwave source and/or its power supply.

According to another aspect of the invention, a microwave coupling for a coaxial line comprises a coupling face located generally at an end of a coaxial line and electrically conductively coupled to the inner conductor of the line, and a lamp support extending from the end of the coaxial line and adapted to support an ultraviolet electrodeless discharge lamp in abutment with the coupling face such that in use, microwave energy is coupled from the coupling face to the lamp.

Preferably the coupling face is integral with the inner conductor of the line. Preferably also, the lamp support is integral with the outer conductor of the line.

In the preferred embodiment, the inner diameter of the outer conductor and the outer diameter of the inner conductor are 34 and 15 mm respectively which with an air dielectric provides an impedance of approximately 50 ohms for the coaxial line. This impedance is well matched to many commercial magnetrons. Furthermore, the outer dimension of 15 mm for the inner conductor is a suitable dimension for the diameter of a cylindrical electrodeless discharge lamp. Thus, the coupling may be manufactured by terminating the outer conductor of a coaxial line at a point in the axial direction of the line beyond the termination of the inner conductor. Thereby, the lamp support is formed at least in part by a portion of the outer conductor of the coaxial line which extends beyond the coupling face in the axial direction of the line to form the sides of a support recess dimensioned to receive an ultraviolet electrodeless discharge lamp. In this arrangement, preferably the coupling face forms the base of the support recess.

In the absence of a bulb or when the bulb has not struck, the coaxial line forms a circular pipe (rather than a transmission line) which has dimensions beyond the cut-off point of the microwave energy. Thus very little microwave energy is radiated from the end of the coaxial line. Leakage in these circumstances is reduced as the distance between the end of the outer conductor (or an electrically conductive material coupled to it) and the end of the inner conductor or coupling face is increased. Thus the invention may be arranged such that the sides of the recess include a transmissive portion which is substantially transmissive to ultraviolet radiation and substantially opaque to microwave radiation. Preferably the transmissive portion is constructed from an electrically conductive reticular material and is electrically coupled to the outer conductor of the line. This may for example be a tube of mesh-like material which is coupled to the outer conductor and extends over the bulb.

The sides of the recess may include a reflective portion which is substantially reflective to ultraviolet radiation and substantially opaque to microwave radiation and which is electrically coupled to the outer conductor of the line. This reflective portion may be arranged to form a focusing reflector. Thus, for example, a paraboloid reflector may be formed as an extension of the outer conductor and pass along one side of the lamp. This arrangement is suitable where the lamp is used in a horizontal configuration. The reflector may be formed from polished aluminium or any other suitable material.

A reflective portion may instead be formed from a material which is not microwave opaque.

Depending on the dimensions of the lamp and on the amount to which it has been inserted into the recess of the coupling of the above described microwave coupling and/or into a microwave cavity or waveguide forming part of the above described apparatus, the intensity of ultraviolet emission may vary throughout the volume of the bulb. It is believed that this is due to variations in microwave field intensity through the volume of the bulb resulting from only a portion of the bulb being exposed to microwave energy or from standing waves created by reflections of microwave energy caused by the electrical discontinuity of the end of the exposed tube.

Another aspect of the invention is an electrodeless discharge lamp including an antenna couplable to the coupling face and arranged to re-radiate into the lamp microwave energy received from the coupling portion to equalise the intensity of ultraviolet emission through the volume of the lamp.

Preferably the antenna includes an elongate conductive portion located within the lamp envelope. With the dimensions given above, a straight 70 mm length of tungsten wire inserted into a bulb of about 180 mm length which in turn has been inserted into a cavity or into recess of the coupling portion described above by about 20 mm is effective.

The elongate portion may have a length which is multiple of λ/4 where λ is the operating wavelength of the lamp i.e. the actual or intended wavelength of the microwave radiation incident on the lamp.

Typically the antenna includes a helical winding extending within and/or without the lamp envelope along at least a part of the lamp envelope. The winding pitch of the helical winding may be between λ/3 and λ/4 and the winding advantageously has a diameter of substantially λ/4.

The invention will now be described by way of example with reference to the drawings in which:-
Figure 1 shows an end elevation of apparatus constructed in accordance with the invention;
Figure 2 is a perspective view of the apparatus of Figure 1;
Figures 3A, 3B, 3C and 3D are partial cut away views of a coupling constructed in accordance with the invention; and
Figures 4A, 4B, 4C and 4D are views of electrodeless discharge lamps in accordance with the invention.

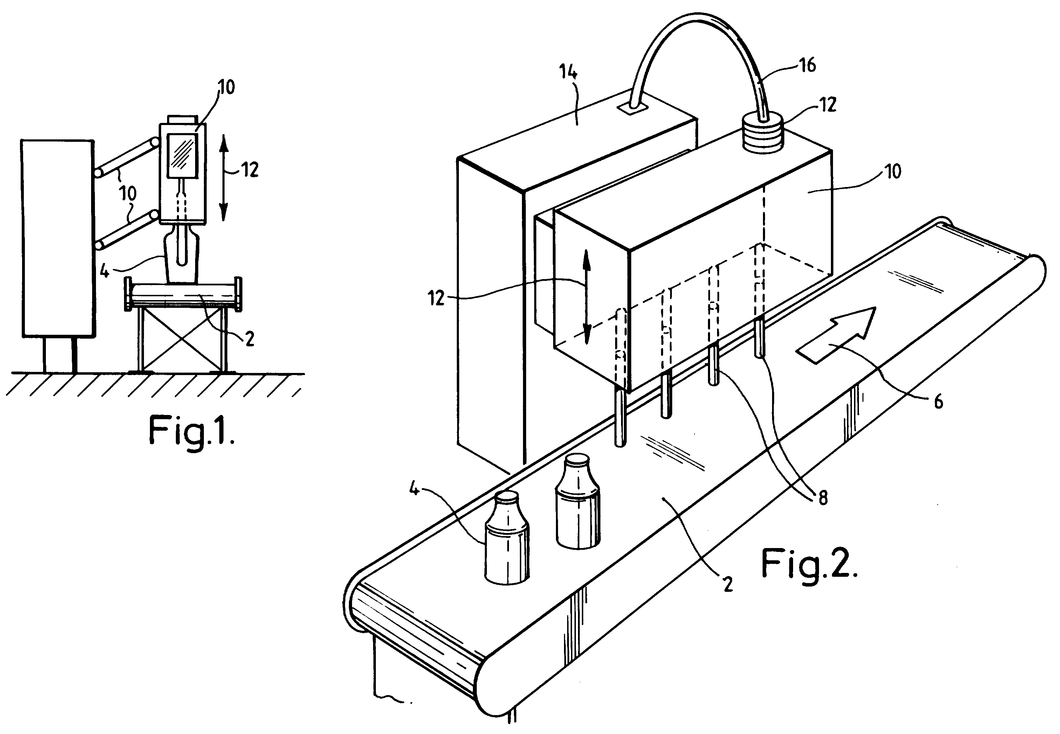

With reference to Figures 1 and 2, apparatus for sterilising containers using ultraviolet radiation on a batch or continuous process is shown.

A conveyor belt 2 carries containers to be sterilised 4 in the direction of arrow 6. The containers 4 are moved past a row of electrodeless discharge lamps 8 which are operable to emit ultraviolet radiation with sufficient intensity to sterilise the interior of the containers 4.

In the preferred embodiment, the bulbs are mounted in the lower surface of a microwave standing wave cavity 10. The cavity is coupled to a magnetron 12 which generates a microwave field within the cavity.

The lamps 8 are mounted with their axes generally vertical and the cavity is driven by arms 10 reciprocally in a generally vertical direction as indicated by arrows 12.

To facilitate the reciprocal movement of the cavity 10, the magnetron 12 is coupled to a power supply unit 14 using a flexible cable 16.

As will be seen, the lamps 8 are only partially inserted into the cavity 10. The majority of the lamps extend vertically below the cavity and the arms 10 are arranged to be driven in synchronism with the passage of the containers beneath the cavity such that the lamps 8 are placed into the containers 4 during their passage along the conveyor 2.

The magnetron 12 may operate continuously so that the bulbs emit ultraviolet light constantly and at a continuous intensity. This may necessitate the conveyer 2 stopping whilst the lamps 8 are inserted into the containers and held during sterilisation. Alternatively, higher power levels may be used which permit the conveyer 2 to move continuously since the time for which the lamps 8 must be inserted into the containers 4 will be shortened by the increased ultraviolet intensity.

The arms 10 may be driven using pneumatic, hydraulic or electrical means in a manner known to the person skilled in the art. Similarly, synchronism of the containers with the reciprocal movement of the cavity 10 and detection of containers which have fallen over etc. may be performed using conventional means such as optical and/or micro switch detectors.

As an alternative to the insertion of the lamps 8 into a cavity 10, each of the lamps may be inserted through the wall of a respective waveguide or more preferably into the end of a respective coaxial line as described below. The waveguides or coaxial lines may be fed from a common microwave source. Instead, several sources may feed one or more cavity, waveguide or coaxial line.

These alternatives have the advantage that a bulky microwave cavity is not required and if the coaxial line is flexible, it also permits the microwave source for example a magnetron, to be statically mounted. This improves the reliability of the magnetron and reduces the mass of the reciprocating parts of the apparatus thereby improving the mechanical response times of the arms 10 to requests to raise and lower the lamps 8.

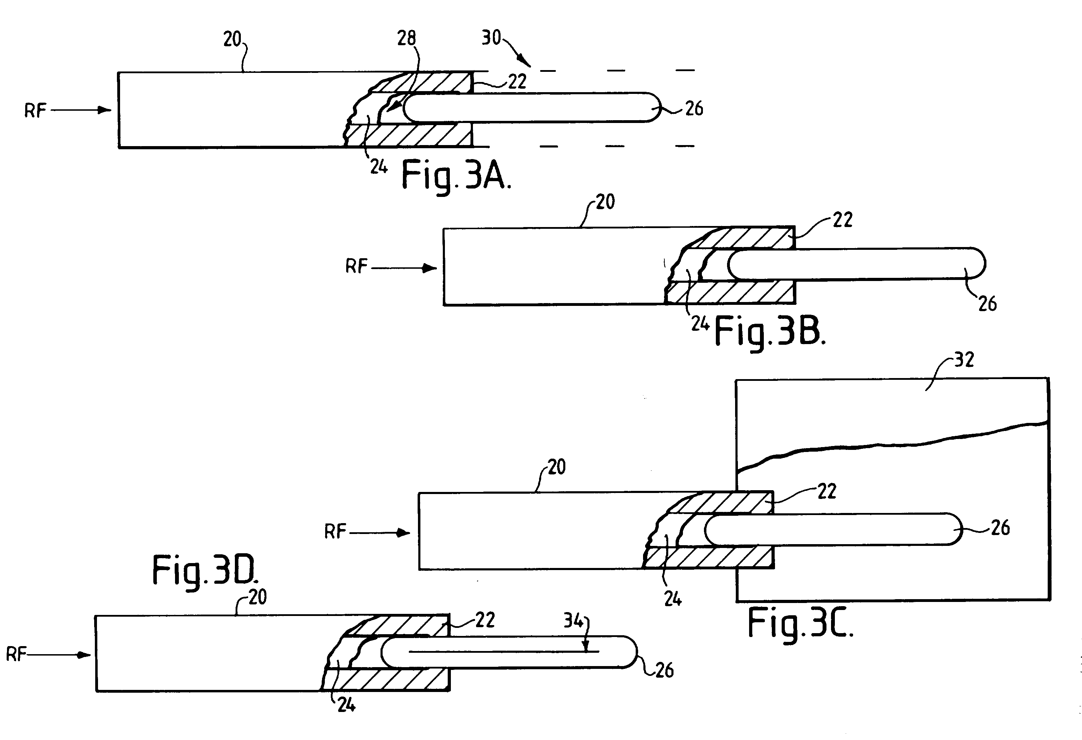

With reference to Figure 3A, a coaxial line 20 is coupled to a microwave source such as a magnetron. The coupling to the magnetron may be achieved in a conventional manner. For example, where the antenna of the magnetron has a ceramic dome covering its antenna, a small section of waveguide may be connected to the magnetron and the outer conductor of the coaxial line may be connected to the wall of the waveguide whilst the inner conductor may extend into the waveguide with a door knob transition. Where the magnetron has an antenna which is uncovered or which is covered with a metallic dome the outer conductor may be coupled to the casing of the magnetron and the inner conductor coupled directly to the antenna or metallic dome. Details of these types of couplings are described in sections 6.13 to 6.15 of "Microwave Transmission Circuits" published by McGraw & Hill in 1948 as part of the Radiation Laboratory Series produced by the Massachusetts Institute of Technology in Cambridge, Massachusetts.

On the right side of Figure 3A, the outer conductor 22 is shown in partial cross-section. The inner conductor 24 has been cut back to allow an electrodeless discharge lamp 26 to be inserted inside the outer conductor.

When microwave energy is passed along the line 20, the lamp 26 is caused to illuminate. The lamp forms a continuation of the centre conductor of the line and when illuminated the lamp 26 forms a lossy antenna in which some or most of the high frequency energy passing along the line is absorbed.

In the preferred embodiment, the outer part of the line 22 extends beyond the inner 24 to prevent RF leakage when the lamp 26 does not light. This is because when the lamp does not light, the coaxial line appears as a circular pipe dimensioned to be beyond the cut off point of the microwave energy. This causes the microwave field to attenuate significantly beyond the point 28 where the inner conductor 24 is truncated.

Figure 3B shows the basic arrangement whereas Figure 3A includes a mesh 30 which is electrically coupled to the outer conductor 28 and forms an electrical extension of the outer conductor to further enhance the prevention of leakage when the lamp 26 does not light. Mesh is used so that very little of the ultraviolet radiation produced by the lamp 26 is obscured.

Figure 3C shows an arrangement similar to that of Figure 3A and 3B but including a reflector 32 to reflect the ultraviolet radiation produced by the lamp 26 and preferably to focus the ultraviolet radiation. This arrangement has particular application in the ultraviolet curing industry where the flexible positioning of the microwave source allows curing equipment to be installed on site more easily. Additionally, in the prior art arrangement, the reflector forms part of a microwave cavity which is completed by an electrically conductive mesh to allow ultraviolet radiation to pass through (but which contains the microwave radiation) and a magnetron is mounted to the back of the reflector to feed the cavity with microwave energy. The present arrangement is less bulky and more efficient. These advantages are attributable also to the other arrangements set out herein.

In Figure 3D, the lamp 26 includes an antenna 34 formed by a length of conductor (preferably tungsten wire of less than 1mm diameter) located within the envelope of the lamp 26. A disadvantage of coupling microwave energy into the lamp 26 in the manner of Figures 3A to 3C is that depending on the dimensions of the line, the degree of insertion of the lamp, into the line and the dimensions of the lamp variations in intensity of ultraviolet emission may occur along the length of the lamp. To overcome this an antenna of the type shown in Figure 3D causes a redistribution of the microwave field within the lamp and tends to equalise the intensity of ultraviolet emissions throughout the volume of the bulb.

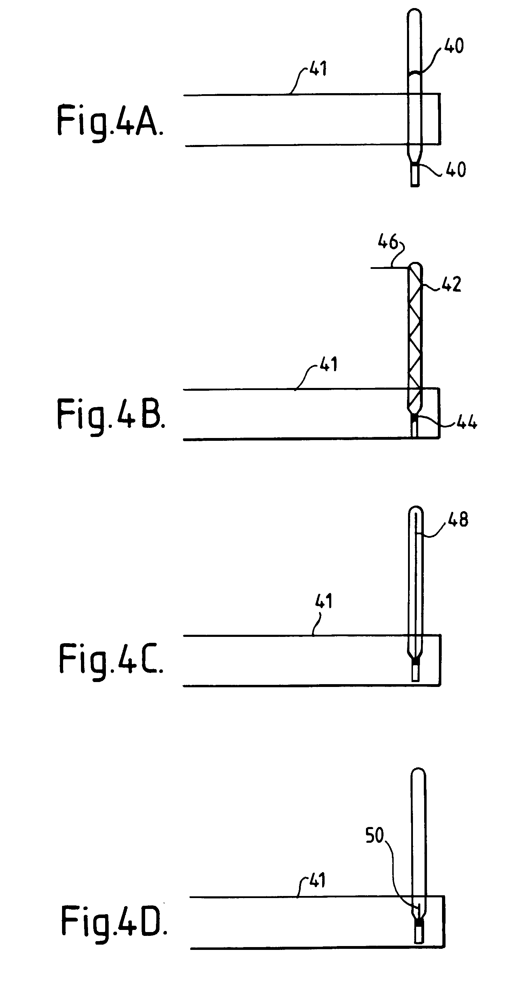

Figures 4A to 4D show alternative antenna arrangements. These are particularly directed to the problems of even illumination when part of a lamp is inserted into a waveguide or standing wave cavity. The principles are applicable also to a lamp coupled directly to a coaxial line. In that configuration, in the embodiment in which the antenna is outside the lamp envelope, the antenna is electrically connected to the inner conductor of the coaxial line.

Referring to Figure 4A, a plain (prior art tube) is inserted into the wall of a waveguide 41. In the absence of an antenna, the bulb is illuminated in an area only within and close to the waveguide as shown by lines 40 through the bulb. The area towards the top of the bulb is not illuminated.

With reference to Figure 4B, a helical winding 42 of.5 mm diameter tungsten wire has been applied to the outer surface of the bulb envelope at a pitch of about 30 mm i.e of about λ/4. The bulb is about 120 mm long and has an approximately 10 mm diameter. At the base of the bulb is a metal section 44 electrically connected to the antenna 42 which forms a door knob transition for coupling into the microwave energy in the waveguide. Figure 4B shows also a quarter-wave antenna 46 at the upper end of the lamp which serves to terminate the winding 42 to provide an even intensity of ultraviolet emission. Without the termination 46 a standing wave will be set up in the winding 42 which causes variations in the brightness of illumination corresponding to the varying energy levels in the winding.

With reference to Figure 4C an alternative antenna in the form of an elongate member extending through the central longitudinal axis of the lamp is shown. Given the dimensions mentioned above, the antenna is of approximately a wavelength in length and there is therefore a bright area in the centre (at approximately a half wavelength position) corresponding to a node of the standing waves set up in the antenna 48. Again this can be adjusted with suitable one or more matched terminations.

With reference to Figure 4D, a uniform illumination may be achieved using a relatively short elongate antenna 50 of less than a quarter-wavelength length.

It will be appreciated that the door knob transition may be replaced with other suitable transitions and furthermore that the bulb need not be of elongate configuration. In particular the bulb may have an annular cross-section and may be in the form of a tube having hollow walls which contain the gaseous charge.

In the embodiment of the lamp described in connection with Figure 4, the antenna may include at least one matched load operable to adjust the standing wave characteristics of the antenna in order to provide generally even emission along the length of the lamp. The matched load may for example be another waveguide (coupled via another transition, for example) or another microwave source. Thus for an elongate lamp, energy may be fed to the lamp from both ends via two waveguides. The antenna may have one or more receiving elements such as the door knob transition described above so that power may be fed from one or magnetrons or may be fed to the lamp from several different positions in relation to the lamp's envelope. Energy may also be fed to both ends of a lamp using the coaxial or microwave cavity embodiments described above.

If it is desired to use different dimension bulbs with the configurations of Figures 3 or 4 which necessitate differently dimensioned coaxial or waveguide couplings, the dimensions of the coaxial line or waveguide may be varied using a tapered or stepped transition over at least the length of one wavelength. Thus larger or smaller diameter bulbs may be used for increased intensity or for very small containers respectively.

Thus, in summary, there has been described apparatus suitable for the continuous sterilisation of articles (preferably their internal surfaces) or to ultraviolet curing processes using an electrodeless discharge lamp energised by microwave energy and operable to emit ultraviolet radiation. A coupling has also been described to permit an electrodeless discharge lamp to be directly coupled to a coaxial line carrying microwave energy. Furthermore, a modified electrodeless discharge lamp has been described which overcomes the problems of emission intensity variations associated with introducing microwave energy only to a portion of such a discharge lamp.

## Claims

1. A microwave coupling for a coaxial line comprising:-
a coupling face located generally at an end of a coaxial line and electrically conductively coupled to the inner conductor of the line, and
a lamp support extending from the end of the coaxial line and adapted to support an ultraviolet electrodeless discharge lamp in abutment with the coupling face such that in use, microwave energy is coupled from the coupling face to the lamp.

2. A coupling according to claim 1, wherein the coupling face is integral with the inner conductor of the line.

3. A coupling according to claim 1 or claim 2, wherein the lamp support is integral with the outer conductor of the line.

4. A coupling according to any preceding claim, wherein the lamp support is formed at least in part by a portion of the outer conductor of the coaxial line which extends beyond the coupling face in the axial direction of the line to form the sides of a support recess dimensioned to receive an ultraviolet electrodeless discharge lamp.

5. A coupling according to claim 4, wherein the coupling face forms the base of the support recess.

6. A coupling according to claim 4 or claim 5, wherein the sides of the recess include a transmissive portion which is substantially transmissive to ultraviolet radiation and substantially opaque to microwave radiation.

7. A coupling according to claim 6 wherein the transmissive portion is constructed from an electrically conductive reticular material and is electrically coupled to the outer conductor of the line.

8. A coupling according to any of claims 4 to 7, wherein the sides of the recess include a reflective portion which is substantially reflective to ultraviolet radiation and substantially opaque to microwave radiation and which is electrically coupled to the outer conductor of the line.

9. A coupling according to claim 9 wherein the reflective portion is arranged to form a focusing reflector.

10. An electrodeless discharge lamp including an antenna, couplable to the coupling face of any preceding claim or for partial insertion into a microwave cavity or waveguide and arranged to re-radiate into the lamp microwave energy incident on the lamp to equalise the intensity of ultraviolet emission through the volume of the lamp, the antenna including an elongate conductive portion located within the lamp envelope and wherein the elongate portion has a length which is a multiple of λ/4, where λ is the operating wavelength of the lamp.

11. An electrodeless discharge lamp including an antenna couplable to the coupling face of any preceding claim or for partial insertion into a microwave cavity or waveguide and arranged to re-radiate into the lamp microwave energy incident on the lamp to equalise the intensity of ultraviolet emission through the volume of the lamp, the antenna including a generally helical winding extending within and/or without the lamp envelope along at least a part of the lamp envelope.

12. A lamp according to claim 11, wherein the winding pitch of the helical winding is between λ/3 and λ/4.

13. A lamp according to claim 11 or claim 12, wherein the winding has a diameter of substantially λ/4.

14. Ultraviolet irradiating apparatus comprising a microwave source, an ultraviolet electrodeless discharge lamp and conveyor means for conveying articles to be irradiated along an irradiating path through the apparatus on a continuous basis, the lamp being mounted such that reciprocal movement of the conveyor means and/or the lamp relative to one another permit the lamp to be inserted and removed from articles passing along the irradiating path thereby to irradiate the articles with ultraviolet radiation emitted from the lamp.

15. Apparatus according to claim 14, wherein the apparatus is arranged such that the articles are not exposed to significant levels of microwave energy.

16. Apparatus according to claim 14 or claim 15, including the microwave coupling of any of claims 1 to 9.

17. Apparatus according to any of claims 14 to 16, including the lamp of any of claims 10 to 13.

18. A method of energising an ultraviolet emitting electrodeless discharge lamp comprising exposing a portion of the lamp to microwave radiation.

19. A method according to claim 18, wherein the lamp is exposed to microwave radiation by partial insertion into a microwave cavity, a waveguide carrying microwave energy or by direct coupling to a coaxial line.
